# EUROPEAN PATENT SPECIFICATION

(11) **EP 2 555 844 B1**
(45) Date of publication and mention of the grant of the patent: **06.08.2014**
(21) Application number: 11762075.7
(22) Date of filing: 29.03.2011
(51) Int. Cl.: B01D 15/18, C13K 13/00, C13B 35/06, C07C 229/12

(54) **SEPARATION PROCESS**
SEPARATIONSVERFAHREN
PROCÉDÉ DE SÉPARATION

(30) Priority: 28.05.2010 US 349307 P; 30.03.2010 US 318950 P
(43) Date of publication of application: 13.02.2013
(73) Proprietor: DuPont Nutrition Biosciences ApS, 1001 Copenhagen K (DK)
(72) Inventor: AIRAKSINEN, Jyrki, FI-02760 Espoo (FI); PAANANEN, Hannu, FI-02460 Kantvik (FI); LEWANDOWSKI, Jari, FI-02580 Siuntio (FI); LAIHO, Kari, FI-02400 Kirkkonummi (FI)
(74) Representative: Kolster Oy Ab
(86) International application number: PCT/FI2011/050264
(87) International publication number: WO 2011/121181

(56) References cited:
- WO-A1-97/45185
- WO-A1-97/45185
- US-A1- 2001 009 236
- US-A1- 2001 009 236
- US-B1- 6 224 776
- US-B1- 6 224 776
- US-B2- 6 875 349
- US-B2- 6 875 349

## Description

### Field of the invention

The invention relates to the field of chromatographic separation and especially to chromatographic fractionation of solutions containing sugars, sugar alcohols, sugar acids and/or betaine by chromatographic sequential multiprofile simulated moving bed (SMB) methods. The solutions are typically multicomponent plant-based extracts and hydrolysates and derivatives thereof, such as sugar beet based solutions or xylose-containing plant hydrolysates. The multiprofile SMB process of the invention aims at improving the separation capacity of the process while maintaining or even improving the yield and/or the purity of the products and/or the water to feed ratio of the system.

### Background of the invention

US 6 875 349 B2, US 6 572 775 B2 and US 6 572 776 B2 disclose a method and a system for fractionating a solution into two or more fractions by a chromatographic simulated moving bed (SMB) process, wherein the separation system comprises at least two separation profiles in the same loop. The SMB process may be continuous or sequential. It is recited that the two profiles are formed by adding at least two portions of the feed solution to the system before recovering product fractions therefrom (column 5, lines 9 to 11). It is also recited that water may be used as an eluent and that water can be added between or after the feed solution additions to ensure no overlap of the two separation profiles (column 5, lines 11 to 14). It is also recited that there can be two or three parallel operations within one separation step (column 6, lines 7 to 8). Furthermore, it is recited that the two or more profiles are moving in the entire resin bed (all columns in the loop) (column 1, lines 32 to 34), i.e. said at least two separation profiles are present in a loop formed by all columns.

Suitable raw materials for the fractionation in the processes of the above-mentioned US patents may be molasses, vinasse, fructose/glucose syrups, beet-derived juices and wood hydrolysates, for example. It is recited that highly preferred raw materials include molasses, vinasse and sulphite cooking liquor. The products that are recovered include for example glucose, fructose, sucrose, betaine, inositol, mannitol, glycerol, xylitol, xylose, sorbitol and erythritol.

The examples of the above-mentioned US patents disclose a two-profile separation of various raw materials, such as molasses, vinasse, glucose/fructose mixtures and xylitol run-off. For example, Example 1 of US 6 875 349 B2 discloses two-profile sequential SMB separation of molasses in a separation system comprising two columns (with eight separate partial packed beds altogether). The fractionation is performed in an eight-step sequence with a sequence length of 38 minutes. Molasses used as the starting material contains 57.6% sucrose and 7.6% betaine on DS. The fractionation provides a sucrose fraction with a purity of 90.1 % by weight and a betaine fraction with a purity of 43.1 % by weight.

Example 4 of US 6 875 349 B2 discloses three-profile sequential SMB separation of molasses in a separation system comprising three separate columns. The fractionation is performed in an eight-step sequence with a sequence length of 43 minutes. Molasses used as the starting material contains 60.4% sucrose and 5.1% betaine on DS. The fractionation provides a sucrose fraction with a purity of 92.7% by weight and a betaine fraction with a purity of 36.6% by weight.

Example 5 of US 6 875 349 B2 discloses two-profile continuous SMB separation of molasses containing 59% sucrose. A sucrose fraction having a purity of 87.8% is obtained.

Furthermore, a further US Patent US 5 466 294 discloses a chromatographic single-profile method for separating sucrose from a soft raw juice obtained from sugar beets.

US 6 093 326 discloses a method for the fractionation of molasses by a chromatographic SMB system, wherein sucrose and betaine are recovered during a multistep separation sequence in two or more loops. Example 3 of the document discloses a process for the fractionation of beet molasses with a step comprising three recycling phases.

However, the multiprofile chromatographic separation methods known from the prior art have for example the disadvantage that the separation capacity, product yield and product purities has not always been at an acceptable level. Consequently, there exists a need for improved multiprofile chromatographic separation methods with increased separation capacity, while essentially maintaining or even improving the yield and the purity of the products.

Furthermore, multiprofile sequential SMB separation processes, especially with a three-profile mode, for recovering sucrose and betaine from sugar beet based solutions having a high sucrose content (more than 70%) are not suggested or disclosed in the art.

### Definitions relating to the invention

"A product fraction" is a fraction taken out of the chromatographic separation process and comprising product components. There can be one or more product fractions.

"A residue fraction" or "a residual fraction" is a fraction which mainly contains components other than the product components, which are recovered. Said other components are typically selected from salts, organic acids and inorganic acids and salts thereof, for example acetic acid, xylonic acid, amino acids, color compounds, glycerol, lignosulfonates, oligosaccharides, etc., depending on the starting material as well as sugars, sugar alcohols and sugar acids other than the product sugars, sugar alcohols and sugar acids. There can be one or more residue fractions. The components of the residual fraction (other than the product components) are also referred to as "residual components". The nature of the residual components depends on the starting material.

"A recycle fraction" is a fraction, which contains incompletely separated product compounds, and which has a lower purity or is more dilute than the product fractions: The recycle fraction is recycled back to the separation to be combined with the feed. The recycle fraction is typically used as diluent of the feed. There may also be one or more operations before returning the recycle to the column(s); for example, the recycle fraction(s) may be concentrated by evaporation. There can be one or more recycle fractions.

"A sequence" or "a separation sequence" is a predetermined sequence of steps which are continuously repeated in a sequential chromatographic separation process, comprising all steps that are required to facilitate the separation of feed components to product fraction(s) and other fractions.

"A step" comprises one or more of a feeding phase, an elution phase, a circulation phase and a PART feeding phase.

During the feeding phase, a feed solution is introduced into a predetermined partial packed bed or predetermined partial packed beds. During the feeding phase, and/or one or more other phases, one or more product fractions and one or more residual fractions can be withdrawn.

During the elution phase, an eluent is fed into predetermined partial packed beds.

The eluent is typically selected from water, ion exchanged water and condensate from evaporation.

During the circulation phase, essentially no feed solution or eluent is supplied to the partial packed beds and no products are withdrawn.

During the part feeding phase, a part of the separation profile is introduced back to the separation as a substitute of eluent. The part feeding phase is present when applying the methods of WO 2010/097510 A1 (US 2010-0213130 A1) and WO 2010/097511 A1 (US 2010-0212662 A1) to the multiprofile method of the invention. The whole content of the recited documents is incorporated herein by reference.

"SMB" refers to a simulated moving bed system.

In a sequential SMB system, not all of the fluid streams (the supply of a feed solution and an eluent or PART, circulation of the separation profile, and withdrawal of the products, recycle or PART) flow continuously.

"A feed" is an amount of feed solution introduced into the separation column during one sequence.

"A subprofile" is a concentration profile of one component, also named as the component peak.

"A separation profile" refers to a dry substance profile formed from the dissolved substances (DS) present in the feed on account of feeding of eluent and the feed solution and flowing through the resin bed, obtained by accomplishing/repeating the separation sequence.

"A part of the separation profile" (equal to "part" or "PART") refers to any section of the separation profile which contains liquid and components in this section and which is used as eluent replacement.

"A part feeding phase" refers to the introduction of the part into the separation system as an eluent replacement.

"A retention volume" (Rt) is the volume of the mobile phase required to elute a component or a certain point of the separation profile through a resin bed. The retention volume of a component may be expressed as % of the resin bed volume. In connection with the present invention, a retention volume especially refers to the volume required to elute the start of a product component fraction (such as a xylose, fructose, betaine or maltose product fraction) through the column.

"Tailing" refers to the phenomenon in which the normal Gaussian peak has an asymmetry factor >1. Tailing is most often caused by sites on the packing that have a stronger than normal retention for the solute.

"A void" or "void volume" in connection with the present invention refers to the volume required to elute the start of the conductivity peak (salts) through the column.

"BV" refers to the resin bed volume of columns, partial packed beds or a separation system.

"Peak broadening" refers to the dispersion of a chromatographic peak (separation profile) as it moves through the column.

"Volume of steps" (V) refers to the volume of the mobile phase (including the feed, eluent and circulation), which moves a component, a separation profile or parts thereof through the separation column(s) from a predetermined step in a separation sequence to another predetermined step in the same or following sequences. The volume of steps is calculated step by step by summing up the volumes of the mobile phase transferred in each step (the volume introduced into the columns in each step during the feeding, elution and/or circulation phases and during the optional part feeding phase).

"An eluent introduction position" refers to any location in the chromatograhic system where eluent may be introduced.

"DS" refers to the dissolved dry substance content. Equal to "dissolved solids content".

"Purity of a component" refers to the content of the component on DS.

"Separation capacity" refers to the amount of a product (kg of dry substance)/volume of separation resin (m³)/hour (h). Recycle and parts introduced in the part feeding phase are not included.

"The W/F ratio" refers to the ratio of the volume of eluent water to the volume of the feed.

### Brief description of the invention

The present invention relates to a process for separating and recovering products such as sugars, sugar alcohols, sugar acids, organic acids and betaine from plant based feed solutions by a chromatographic multiprofile sequential simulated moving bed (SMB) system. The invention is based on recovering product fractions and/or recycle fractions from several positions of the multiprofile sequential SMB chromatographic separation system. In practice, product fractions and/or recycle fractions are collected from several columns of the chromatographic separation system. The objects of the invention are achieved by a process which is characterized by what is stated in the independent claims. Preferred embodiments of the invention are disclosed in the dependent claims.

Surprisingly it was found out that the process of the invention provided improved separation capacities, product yields, and product purities compared to the multiprofile separation systems known from the prior art.

### Description of the invention

The invention relates to a process of separating and recovering at least one product from a feed solution containing at least one product compound selected from sugars, sugar alcohols, sugar acids, organic acids and betaine, whereby the process is carried out in a chromatographic sequential simulated moving bed (SMB) system, which comprises a plurality of columns containing one or more partial packed beds, wherein the columns form one or more loops, comprising

creating three successive separation profiles in the system by repeating a predetermined separation sequence, whereby said three successive separation profiles are simultaneously present in the system, and each separation profile comprises at least one product subprofile, a residual subprofile and optionally other subprofiles,

moving said three separation profiles forward through the system by repeating the predetermined separation sequence, and

recovering at least one product fraction enriched in one product compound from a column and also recovering at least one further fraction from one or more other columns of the system, whereby said at least one further fraction is a further product fraction enriched in the same product compound and/or a recycle fraction containing the same product compound.

In the process of the invention, the product components may be selected from sugars, sugar alcohols, sugar acids, organic acids and betaine.

The sugars may be selected from xylose, fructose, glucose, mannose, arabinose, galactose, rhamnose, fucose, sucrose, raffinose, maltose, levoglucosan, ribose, isomaltulose, tagatose, trehalose, trehalulose, and psicose.

In connection with the present invention, sugars also comprise oligomeric compounds, such as xylo-oligosaccharides, maltooligosaccharides, fructo-oligosaccharides, and polydextrose.

The sugar alcohols may be selected from xylitol, mannitol, sorbitol, inositol, maltitol, isomalt, glycerol and erythritol.

The sugar acids may be selected from hydroxy acids, carboxylic acids, such as aldonic acids, e.g. xylonic acid, gluconic acid, and itaconic acid, and uronic acids, such as glucuronic acid and galacturonic acid, for example.

The organic acids may be selected for example from amino acids, such as glutamic acid.

Especially preferred product components in connection with the present invention are sucrose, betaine and xylose.

The residual components are typically selected from salts, inorganic acids and salts thereof, e.g. sulfuric acid and sulfurous acid, amino acids, color compounds, glycerol, lignosulfonates, oligosaccharides, etc., depending on the starting material as well as sugars, sugar alcohols, sugar acids and organic acids other than the product sugars, sugar alcohols, sugar acids and organic acids.

For example, in the separation of sugars, sugar alcohols, and sugar acids from plant-based hydrolysates, such as sulfite spent liquor, the residual components mainly comprise lignosulfonates, oligosaccharides, salts, organic acids (e.g. acetic acid and xylonic acid), and inorganic acids, etc. In the separation of betaine from sugar-beet based solutions, such as molasses and vinasse, the residual components mainly comprise salts, color compounds, organic acids and salts thereof, amino acids, glycerol, and mono-, diand trisaccharides, etc. In the separation of cane molasses, the residual components mainly comprise salts, color compounds, mono-, di- and trisaccharides.

The starting materials containing one or more product components selected from sugars, sugar alcohols, sugar acids, organic acids and betaine are typically multicomponent plant-based extracts and hydrolysates or derivatives thereof. Sulfite spent liquor, starch hydrolysate, and sugar beet based solutions, such as low green, molasses, raw juice, thick juice, stillages and fermentation products thereof, such as vinasse, can be mentioned as examples of suitable starting materials.

One embodiment of the invention comprises the separation of sugars, sugar alcohols, and sugar acids from plant-based hydrolysates, plant-based extracts and derivatives thereof. The plant-based hydrolysates may be obtained from vegetable material, including wood material from various wood species, particularly hardwood, such as birch, aspen and beech, maple, eucalyptus, various parts of grain (such as straw, especially wheat straw, husks, particularly corn and barley husks and corn cobs and corn fibers), bagasse, cocoanut shells, cottonseed skins, almond shells, etc. The plant-based extracts may be for example water, vapour, alkaline or alcohol extracts of the plants described above. Derivatives of plant-based hydrolysates and extracts may be different post-treated products, such as evaporation products thereof or fractions from membrane processes.

In one specific embodiment of the invention, the plant-based hydrolysate for the separation of sugars, sugar alcohols, and sugar acids, such as xylose, is a spent liquor obtained from a pulping process. A typical spent liquor useful in the present invention is a spent sulfite pulping liquor, which is preferably obtained from acid sulfite pulping. One example of a useful hydrolysate is a prehydrolysate from sulfate pulping.

In one embodiment of the invention, xylose is separated from a wood hydrolysate, such as a spent sulphite pulping liquor.

In a further specific embodiment, xylose is separated from a xylose run-off obtained from the crystallization of xylose. In a further specific example of the invention, xylitol is separated from a xylitol run-off after hydrogenation of xylose and crystallization of xylitol.

In a further specific embodiment, xylitol or erythritol is separated from fermentation broths.

A still further embodiment of the invention comprises the separation of betaine from a sugar beet based solution, such as low green, molasses and vinasse.

In the present invention, said low green refers to a sugar beet based syrup containing 71 to 85% sucrose and 2 to 10% betaine on DS. Furthermore, the low green contains other components (for example salts, color compounds, organic acids, amino acids etc.) in a typical amount of 13 to 27% on DS. A typical example of a useful starting material in the present invention is a low green containing about 72% sucrose, 3 to 6% betaine and 20 to 25% ash (salts) and other inorganic/organic components.

Another embodiment of the invention comprises the separation of fructose from mixtures of glucose and fructose, such as solutions of inverted sucrose, solutions of isomerized glucose and mixtures thereof as well as run-offs obtained from the crystallization of fructose.

A further embodiment of the invention comprises the separation of maltose from starch hydrolysates, such as maltose syrups. A still further embodiment of the invention comprises the separation of maltitol from a maltitol syrup after hydrogenation of maltose.

In preferred embodiments of the invention, the feed solution is selected from plant-based hydrolysates and extracts, fructose/glucose syrups, invert sugar mixtures and starch hydrolysates. In an especially preferred embodiment of the invention, the plant-based hydrolysates and extracts are selected from sugar beet based solutions and wood hydrolysates. The sugar beet based solutions are especially selected from low green, molasses, thick juice and raw juice and betaine-containing fractions thereof. The wood hydrolysates are especially selected from spent sulphite pulping liquor.

The chromatographic separation system of the present invention comprises a plurality of columns, which refers to more than one columns. In a typical embodiment of the invention, the system comprises three or more separation columns containing one or more partial packed beds. In a preferred embodiment of the invention, the system comprises at least three columns, for example 3 to 12 columns, preferably 6 to 9 columns.

In a typical embodiment of the invention, the columns have an equal size. The columns may also have a different size in relation to each other. The columns may also be divided into two or more compartments.

The chromatographic separation resins in the partial packed beds of the separation system may be selected from those commonly used for the separation of the above-described product components from multicomponent plant-based extracts and hydrolysates and derivatives thereof. Especially useful resins are strongly acid cation exchange resins (SAC) and weakly acid cation exchange resins (WAC), but even weakly basic anion exchange resins (WBA) and strongly basic anion exchange resins (SBA) can be used. The cation exchange resins (SAC and WAC) may be in monovalent, divalent or trivalent form, such as in H⁺, Na⁺, K⁺, Ca²⁺, Mg²⁺, Zn²⁺, Sr²⁺ , Ba²⁺ or Al³⁺ form.

The resins may be styrenic or acrylic resins having a crosslinking degree in a range of 1 to 20%, for example 4 to 10% DVB (divinylbenzene), preferably 4.5 to 7.5% DVB. The crosslinking degree of the resins as a rule affects the retention volume of the components. A typical mean particle size of the resins is 200 to 450 µm.

The columns/partial packed beds of the system form one or more loops. In a preferred embodiment of the invention, the system comprises a loop formed by all columns of the system in one or more steps of the separation sequence, i.e. there is a loop from the last column to the first column of the system.

A loop may be closed or "open". In a closed loop liquid is circulated and essentially nothing is fed or withdrawn from the loop. In an open loop eluent or feed or PART can be introduced into the loop and a product fraction or a residual fraction or a PART or can be withdrawn therefrom. During the feed, the elution and the part feeding phase, the flow through the packing material beds may take place between successive loops, wherein the flows carry material from one loop to another. During the circulation phase, the loop is closed and separated from the other loops.

The sequential SMB process of the present invention comprises sequences, steps and phases. The sequences, steps and phases are defined above. There can be 1 to 4 parallel same or different phases (selected from feeding, elution and/or circulation and optionally part feeding) in one separation step.

The fractionation is carried out using the sequences, steps and phases above, whereby a separation profile (i.e. a dry substance profile) is formed in the separation columns from the dissolved substances present in the feed. The separation profile comprises at least one product subprofile, a residual subprofile and optionally other subprofiles.

In the present invention, the fractionation is effected by creating three separation profiles by repeating the predetermined separation sequence and moving the separation profiles forward in the SMB system by repeating the sequence. The sequence has to be repeated at least three times to elute the profile through the whole SMB system.

In a preferred embodiment of the invention, said three separation profiles are present in a loop formed by all columns of the system.

In a further embodiment of the invention, the process may comprise a step where the columns form three separate loops and a separation profile is moving forward in each loop, i.e. one separation profile is present in each three loops. For example, columns 1 and 2, columns 3 and 4 as well as columns 5 and 6 in a six-column system may form three separate loops and the separation profile is moving forward preferably simultaneously in each loop. The optional three-loop arrangement facilitates advantageous colour removal to the residual fractions.

Typically the first column of the system is the feed column of the system.

The feed volume is selected so that the columns are able to separate the product compound from other components of the feed with desired yields to provide product fractions with a desired purity. In one embodiment of the invention, the feed volume may be 4.5-10% of the total bed volume.

The feed concentration (dry substance content) is typically from 40 to 65 g dry substance/100 g feed solution and preferably from 45 to 55 g dry substance/100 g feed solution.

The water intervals between successive profiles at the outlet of the columns are minimized, and there may even be overlap between the successive separation profiles, contrary to US 6,875,349 B2. Especially, this concerns the embodiment of the invention, which relates to the separation of sucrose and betaine from sugar beet based solutions. There may be overlap between the betaine subprofile and the residual subprofile of the successive separation profiles.

The three-profile mode of the present invention provides, as a further advantage, short sequence times. In the three-profile mode of the present invention, the sequence time is short (for example 30 minutes) compared to a two-profile mode (for example 47 minutes). Correspondingly a single profile mode would take about two times 47 min, i.e. about 94 minutes.

Finally, the process of the invention comprises recovering at least one product fraction enriched in one product compound from one column and also recovering at least one further fraction from one or more other columns of the system, whereby said at least one further fraction is a further product fraction enriched in the same product compound and/or a recycle fraction containing the same product compound. The product fractions are recovered and withdrawn from the process and preferably combined. The fractions are circulated back to the separation to dilute the feed. The combined volume of the recycle fractions is typically in the range of 5% to 50% of the feed volume.

Said at least one product fraction enriched in one product compound is preferably recovered from the last column of the system.

In the process of the present invention, the last column refers to the last column downstream from the feed column. The feed column is usually the first column of the system.

Said at least one further fraction selected from a further product fraction enriched in the same product compound and a recycle fraction containing the same product compound is recovered from one or more other columns of the system, which are preferably selected from any upstream columns of the system in regard to the column from which said at least one product fraction is recovered.

In one embodiment of the invention, said at least one product fraction enriched in one product compound and said at least one further fraction selected from a further product fraction enriched in the same product compound and a fraction containing the same product compound are recovered from one and the same separation profile during more than one separation sequences.

In another embodiment of the invention, said at least one product fraction enriched in one product compound and said at least one further fraction selected from a further product fraction enriched in the same product compound and a recycle fraction containing the same product compound are recovered from more than one separation profiles during one and the same separation sequence.

Surprisingly, it was found that collecting product fractions and/or fractions from several columns (several positions of the separation system) in accordance with the present invention made it possible to fit three separation profiles into the SMB system.

In a further embodiment of the invention, the process also comprises recovering other fractions, such as residual fractions. This mode of operation facilitates the fitting of three profiles in the SMB system and also facilitates the recovery of the product fractions with high yield and purity. Residual fractions containing essentially no product are withdrawn from a plurality of columns. In other words, the residual is withdrawn from the columns as soon as the desired product is separated therefrom. Consequently, the size of the residual peak/subprofile (salt peak/subprofile) will decrease while the fractionation proceeds, which leads to further reduced fronting of the product peak/subprofile. This mode of operation may be applied for example to the recovery of sucrose and betaine from sugar beet based solutions.

In a further embodiment of the invention, the process further comprises recovering one or more further fractions enriched in at least one further product compound. Said one product compound may be for example sucrose and said further product compound may be for example betaine.

In a further embodiment of the invention, the process comprises recovering at least one sucrose fraction from a column and at least one further sucrose fraction from one or more other columns of the system and also recovering at least one betaine fraction from any columns of the system. In this embodiment of the invention, the process may further comprise recovering at least one recycle fraction containing sucrose from one or more other columns of the system.

In a still further embodiment of the invention, the process comprises recovering at least one xylose fraction from a column and also recovering at least one further xylose fraction and/or at least one fraction containing xylose from one or more other columns of the system.

In one specific embodiment of the invention, the invention relates to a process for recovering sucrose and betaine from a sugar beet based solution in a chromatographic sequential simulated moving bed (SMB) system, which comprises a plurality of columns containing one or more partial packed beds, wherein the columns form one or more loops, comprising

creating three successive separation profiles in the system by repeating a predetermined separation sequence, whereby said three successive separation profiles are simultaneously present in the system, and each separation profile comprises a sucrose subprofile, a betaine subprofile, a residual subprofile and optionally other subprofiles,

moving said three separation profiles forward through the system by repeating the predetermined separation sequence, and

recovering at least one sucrose fraction from a column and at least one further sucrose fraction from one or more other columns of the system and also recovering one or more betaine fractions from any columns of the system.

The sugar beet based solution used as the feed in this embodiment of the invention is preferably selected from low green, molasses, thick juice and raw juice.

It is also possible to recover betaine and salts in the same fraction, especially when there is overlap between the betaine subprofile and the residual subprofile of successive separation profiles. It was found that the overlapping of the betaine subprofile and the residual subprofile of successive separation profiles was also beneficial for fitting three separation profiles into the system.

Furthermore, it was surprisingly found that the fronting of the sucrose peak/subprofile was reduced for the reason that the starting material with a high sucrose purity (>70%) already has a relatively low salt content. Consequently, the sucrose peak/subprofile and the whole separation profile remain advantageously narrow during the fractionation, which further helps to fit three separation profiles in the SMB system.

In this embodiment of the present invention, the fractionation may be effected so that the slow-moving betaine and the fast-moving salts are essentially recovered in separate fractions. It is also possible to recover betaine and salts in the same fraction, whereby there is overlap between the betaine subprofile and the residual subprofile. The fraction containing betaine and residual components may then be divided into several fractions, for example into a fraction enriched in betaine and a fraction enriched in salts and other residual components. Betaine can then be recovered from the betaine fraction separately.

In a further embodiment of the invention, betaine is recovered from the residual fraction or from a separate betaine fraction for example by further chromatographic fractionation, followed by crystallization.

In the embodiment of the invention relating to the separation of betaine and sucrose from sugar beet based solutions, sucrose fractions recovered from separate columns are obtained with a purity (sucrose content) of more than 90%, preferably more than 92% and more preferably more than 94% on the dry substance (DS). The betaine content of the sucrose fraction is less than 0.5%, preferably less than 0.1% on DS.

In this embodiment of the invention, the dry substance amount of said further sucrose fraction recovered is more than 10%, preferably more than 30% and more preferably more than 50%, based on the dry substance amount of the combined sucrose fractions recovered.

In this embodiment of the invention, the process provides into combined sucrose fractions a sucrose yield of more than 90%, preferably more than 92% and more preferably more than 94% based on the sucrose of the feed solution.

In this embodiment of the invention, betaine fractions are obtained with a purity (betaine content) of more than 35%, preferably more than 45% and more preferably more than 55% on DS. In a typical embodiment of the invention, the content of betaine in the betaine fraction is in the range of 35% to 75%, preferably 45 to 75% on DS. The betaine content of the residual fractions is typically in the range of 0.1 to 3.0% on DS.

In this embodiment of the invention, the yield of betaine to the betaine fraction(s) is more than 85%, preferably more than 92% and more preferably more than 94%.

In this embodiment of the invention relating to the separation of betaine and sucrose from sugar beet based solutions, the process of the invention provides a high separation capacity, for example a separation capacity over 55 kg dry substance per hour per m³ resin. The capacity up to 150 dry substance per hour per m³ resin can be reached by using shorter bed lengths and a resin with a bead size of less than 350 µm. Furthermore, the process provides a favorable ratio of eluent water to the feed dry substance, for example about 5 - 10 m³ water per ton dry substance. This corresponds to a W/F ratio (the ratio of the volume of eluent water to the volume of the feed) of 3 to 6 when the feed has dry substance content of about 50%.

In another specific embodiment of the invention, the invention relates to a process for recovering xylose from a plant-based hydrolysate in a chromatographic sequential simulated moving bed system, which comprises a plurality of columns containing one or more partial packed beds, wherein the columns form one or more loops, comprising

creating three successive separation profiles in the system by repeating a predetermined separation sequence, whereby said three successive separation profiles are simultaneously present in the system, and each separation profile comprises a xylose subprofile, a residual subprofile and optionally other subprofiles,

moving said three separation profiles forward through the system by repeating the predetermined separation sequence, and

recovering at least one xylose fraction from a column and also recovering at least one further xylose fraction enriched in xylose and/or at least one fraction containing xylose from one or more other columns of the system.

The plant-based hydrolysate used as the feed in this embodiment of the invention may be for example a hemicellulose hydrolysate, preferably sulphite spent liquor.

In this embodiment of the invention, xylose fraction(s) is obtained with a purity (xylose content) of more than 45%, preferably more than 50% and more preferably more than 55% on DS.

In this embodiment of the invention, the process provides a xylose yield of more than 85%, preferably more than 90% and more preferably more than 93% on the xylose of the feed solution.

In the embodiment of the invention relating to the separation of xylose from xylose-containing plant hydrolysates, the process of the invention also provides a high separation capacity, for example a separation capacity of up to 80 kg dry substance per hour per m³ resin. Furthermore, the process provides a favorable ratio of eluent water to the feed dry substance, for example about 3.3 m³ water per ton dry substance. This corresponds to a W/F ratio (the ratio of the volume of eluent water to the volume of feed) of 2.0 when the feed has dry substance content about 50%.

Consequently the three-profile operation mode of the present invention provides increased separation capacity.

Further advantages, such as significant reduction of the amount of eluent water (from 10 up to 50%) in the separation, can be achieved by applying the multiprofile method of the invention to the methods of WO 2010/097510 A1 and WO 2010/097511 A1, which are incorporated herein by reference. The methods disclosed in these applications generally disclose the introduction of various parts of the separation profile back to the separation as eluent replacement. The reduction of the amount of eluent water leads to lower energy requirement in the evaporation of the separated fractions.

Consequently, in a further embodiment of the invention, the process further comprises introducing one or more parts of said three separation profiles back to one or more eluent introduction positions of the system to substitute a portion of the eluent, wherein said parts comprise components selected from product compounds and residual components.

The volume, introduction position and introduction step of said parts are determined on the basis of the retention volumes of the components of said parts, the volume of the resin bed through which components of the parts pass and the volume of the steps moving the components of said parts from the introduction position to the calculated target withdrawal position of the components during said same or following separation sequences while essentially maintaining the purity of the product fraction(s) and the yield of the product components.

It is essential that the volume, introduction position and introduction step of said parts are determined to be suitable so that the components of said parts at the withdrawal thereof either reach the region of the similar fast moving components of the feed or are retained to be eluted together with the faster moving components of the feed or they allow the fast moving components of the separation profile to reach the slow moving components of said parts.

The retention volumes of the components for each separation system are experimentally determined for the resin beds in use. The retention volumes of the components depend on the divinylbenzene (DVB) content of the resins, for example.

In the separation of xylose from xylose-containing plant hydrolysates with strong acid cation exchange resins in a magnesium form having a DVB content of 6.5%, the retention volume of xylose is approximately 60% (between 57 and 63%) of the resin bed volume. The retention volume of the start of the conductivity peak (salts and large molecules, such as lignosulfonates) in the same sulfite spent liquor separation with the same resins is approximately 28 to 34% of the resin bed volume, which is equal to the void volume of the resin bed. Also the peak broadening phenomenon must be taken into account when calculating the elution volumes, in which different components are eluted from the separation column.

In the separation of betaine and sucrose from sugar beet based solutions with strong acid cation exchange resins in monovalent form having a DVB content of 6.5%, the retention volume of betaine is approximately 70% (between 67 and 73%) of the resin bed volume and the retention volume of sucrose is approximately between 55% and 60% of the resin bed volume. The retention volume of the start of the conductivity peak (salts and large molecules) in the same separation with the same resins is approximately between 28 and 34% of the resin bed volume, which is equal to the void volume of the resin bed.

One example of a suitable part of the separation profile for eluent replacement comprises the overlapping part of the betaine peak and the salt peak. The introduction point is selected so that salts as a fast-moving component pass sucrose in the separation, and betaine and salts can be recovered in adjacent fractions or in the same fraction.

Furthermore, parts of said residual fractions may be returned back to the separation as a substitute of eluent. The generation and moving forward of three separation profiles in the same loop for the separation of different starting materials is illustrated in the following non-limiting Examples 2, 3 and 4. Example 2 discloses a three-profile SMB separation of low green into a sucrose fraction, a betaine fraction, a residual fraction (a salt fraction) and a recycle fraction. Example 3 discloses a three-profile SMB separation of low green into a sucrose fraction, a betaine fraction, a residual fraction (a salt fraction) and a recycle fraction. Example 4 discloses a three-profile SMB separation of Mg²⁺ sulphite spent liquor into a xylose fraction, a recycle fraction and a residual fraction.

Example 1A is a reference example, which discloses the fractionation of a low green by a two-profile two-loop sequential SMB method. Examples 1B and 1C are reference examples, which disclose a three-profile SMB separation of molasses. Examples 1 B and 1C differ from the process of the invention in that product fractions are only collected from one column.

Several advantages can be seen when comparing the reference Example 1A and to Example 2 of the invention relating to the fractionation of low green with an equal sucrose and betaine yield and approximately equal water consumption. In accordance with the three-profile method of the invention, betaine can be enriched into the betaine fraction with a betaine content of 51 % with a very high yield (about 95%). Betaine can be further enriched from the betaine fraction with chromatographic separation. The method also provides a sucrose fraction with a very high sucrose content (94 to 95%) and with a very high yield (at least 93%). The production capacity (calculated as kg dry substance / hour /m³ of the resin) can be increased at least 40% compared to the capacity of the two-profile method of Example 1 (54 kg vs. 38 kg dry substance / hour / m³ of the resin).

Furthermore, it can be seen that reference examples 1 B and 1C provide a very low capacity and a very poor betaine purity and yield, i.e. the betaine purity is only about 23 to 37% on DS and the betaine yield is about 32 to 43%. Examples 1 B and 1C also provide a very low sucrose yield of about 79 to 82%.

### Example 1A

### Two-loop two-profile SMB separation of a low green - reference example

The process equipment includes 6 columns, required piping, a feed pump, recycling pumps, an eluent water pump, heat exchangers, feed and eluent and product tanks, flow control means for the out-coming liquids as well as inlet and product valves for the various process streams. The height of all columns is 4.0 m, the diameter of all columns is 5.15 m, and the first column is made of two compartments with an equal size. The columns are packed with a strong acid gel type cation exchange resin (Dowex monosphere 99K/350) in Na⁺-form. The mean bead size of the resin is 0.35 mm.

Before the separation, the low green is diluted with a recycle fraction (obtained from the separation) and carbonated with sodium carbonate to reduce the calcium level of the solution. Finally, the low green is pre-coat filtered using diatomaceous earth as a filtering aid.

The feed was composed as set forth below, whereby the percentages are given on a dry substance weight basis.

**TABLE E1A-1**

| Composition of the feed | |
|---|---|
| Feed pH | 9.5 |
| Feed dry substance, g/100g | 50.0 |
| Sucrose, % on DS | 72.0 |
| Betaine, % on DS | 4.5 |
| Others, % on DS | 23.5 |

The fractionation is performed by way of a 13-step SMB sequence as set forth below. The feed and the eluent are used at a temperature of 80°C, and an evaporation condensate is used as an eluent.

Step 1: 5.0 m³ of feed solution is pumped into column 1 at a flow rate of 185 m³/h, and a recycle fraction is collected from column 6.

Step 2: 2.6 m³ of feed solution is pumped into column 1 at a flow rate of 140 m³/h, and a residual fraction is collected from the same column. Simultaneously, 3.2 m³ of eluent is pumped into column 2 at a flow rate of 170 m³/h, and a recycle fraction is collected from column 6.

Step 3: 2.0 m³ of feed solution is pumped into column 1 at a flow rate of 110 m³/h, and a residual fraction is collected from the same column. Simultaneously, 2.0 m³ of eluent is pumped into column 2 at a flow rate of 110 m³/h, and a betaine fraction is collected from column 4. Also simultaneously, 3.5 m³ of eluent is pumped into column 5 at a flow rate of 198 m³/h, and a sucrose fraction is collected from column 6.

Step 4: 12.6 m³ of feed solution is pumped into column 1 at a flow rate of 121 m³/h, and a residual fraction is collected from the same column. Simultaneously, 9.7 m³ of eluent is pumped into column 2 at a flow rate of 90 m³/h, and a residual fraction is collected from column 4. Also simultaneously, 23.0 m³ of eluent is pumped into column 5 at a flow rate of 220 m³/h, and a sucrose fraction is collected from column 6.

Step 5: 7.2 m³ of feed solution is pumped into column 1 at a flow rate of 190 m³/h, and a sucrose fraction is collected from column 6.

Step 6: 17.6 m³ is looped at a flow rate of 190 m³/h in a loop formed by all columns. There are two separation profiles in the loop.

Step 7: 4.9 m³ of eluent is pumped into column 3 at a flow rate of 190 m³/h, and a residual fraction is collected from column 2.

Step 8: 8.5 m³ of eluent is pumped into column 1 at a flow rate of 137 m³/h, and a residual fraction is collected from column 2. Simultaneously, 10.9 m³ of eluent is pumped into column 3 at a flow rate of 180 m³/h, and a residual fraction is collected from column 5. Also simultaneously, 14.0 m³ of eluent is pumped into column 6 at a flow rate of 224 m³/h, and a betaine fraction is collected from the same column.

Step 9: 5.0 m³ of eluent is pumped into column 1 at a flow rate of 160 m³/h, and a residual fraction is collected from column 2. Simultaneously, 6.0 m³ of eluent is pumped into column 3 at a flow rate of 190 m³/h, and a betaine fraction is collected from column 6.

Step 10: 16.1 m³ of eluent is pumped into column 1 at a flow rate of 190 m³/h, and a betaine fraction is collected from column 6.

Step 11: 15.0 m³ is looped at a flow rate of 190 m³/h in a loop formed with the second compartment of column 1, column 2 and column 3 (the first compartment of column 1 is excluded from the loop). Simultaneously, 7.0 m³ is looped at a flow rate of 90 m³/h in a loop formed by columns 4, 5 and 6.

Step 12: 11.0 m³ of eluent is pumped into column 1 at a flow rate of 150 m³/h and a residual fraction is collected from column 3. Simultaneously, 14.1 m³ of eluent is pumped into column 4 at a flow rate of 190 m³/h, and a residual fraction is collected from column 6.

Step 13: 20.1 m³ is looped at a flow rate of 200 m³/h in a loop formed with columns 1, 2 and 3. Simultaneously, 19.3 m³ is looped at a flow rate of 195 m³/h in a loop formed by columns 4, 5 and 6.

After equilibration of the system, residual fractions, recycle fractions, sucrose fractions and betaine fractions are collected. The results including HPLC analyses for the combined fractions are set forth in the table below.

**TABLE E1A-2**

| | Combined residual | Combined recycle | Combined sucrose | Combined betaine |
|---|---|---|---|---|
| Volume, m3 | 81.3 | 8.2 | 33.7 | 38.1 |
| Dry substance, weight-% | 3.8 | 19.9 | 30.8 | 3.8 |
| Dry substance, g/100ml | 3.9 | 21.6 | 34.7 | 3.9 |
| Sucrose % on DS | 22.9 | 73.6 | 93.5 | 4.4 |
| Betaine, % on DS | 1.2 | 0.0 | 0.0 | 52.5 |
| Others, % on DS | 75.9 | 26.4 | 6.5 | 43.1 |

The overall yield calculated from these fractions is 93.3% for sucrose and 95.5% for betaine. The separation capacity for the fractionation is 38.4 kg dry substance per hour per m³ of the resin and the ratio of eluent water to the feed dry substance is 7.3 m³ water per ton dry substance. This corresponds to a W/F ratio of 4.5.

### Example 1 B 3-Profile/3-loop chromatographic SMB separation of beet molasses - reference example

The process equipment included three columns connected in series, a feed pump, recycling pumps, an eluent water pump, heat exchangers, flow control means for the out-coming liquids as well as inlet and product valves for the various process streams. The height of all columns was 5 m and the diameter was about 0.2 m. The columns were packed with a strong acid gel type cation exchange resin (Finex V09C) in Na⁺-form. The mean bead size of the resin was about 0.37 mm. The DVB content of the resin was 4.5 %.

Before the separation, molasses was pre-coat filtered after carbonation using diatomaceous earth as a filter aid. The feed was composed as set forth below, whereby the percentages are given on a dry substance weight basis.

**TABLE E1B-1**

| | |
|---|---|
| Composition of the feed | |
| Sucrose, % on DS | 60.4 |
| Betaine, % on DS | 5.1 |
| Oligosaccharides, % on DS | 3.9 |
| Others (mainly salts), % on DS | 30.6 |
| Feed Concentration, g/100ml | 74.9 |

The fractionation was performed in accordance with Example 1 of US 6 572 775 B2 by way of 8-step SMB sequence as set forth below. The aim of the separation was to separate sucrose and betaine contained therein. The feed and the eluent were used at a temperature of 75°C and ion exchanged water was used as an eluent.

Step 1: 9.0 l of feed was pumped to column 2 at a flow rate of 70 l/h, and a recycle 1 fraction was collected from column 1.

Step 2: 11.0 l of feed was pumped to column 2 at a flow rate of 70 l/h, and a sucrose fraction was collected from column 1.

Step 3: 16.0 l of water was pumped to column 2 at a flow rate of 70 l/h, and a sucrose fraction was collected from column 1.

Step 4: 5.5 l of water was pumped to column 2 at a flow rate of 70 l/h, and recycle 2 fraction was collected from column 1.

Step 5: 10.0 l of water was pumped to column 3 at a flow rate of 40 l/h, and a betaine fraction was collected from column 1. Simultaneously, 8.0 l was circulated in the loop formed with column 2 at flow rate of 70 l/h.

Step 6: 5 l of water was pumped to column 1 at a flow rate of 40 l/h, and betaine was collected from column 1. Simultaneously, 5.0 l of water was pumped to column 2 at a flow rate of 70 l/h, and a residual fraction was collected from column 2. Also simultaneously, 5.0 l of water was pumped to column 3 at a flow rate of 75 l/h, and a residual fraction was collected from column 3.

Step 7: 15 l of water was pumped to column 1 at a flow rate of 40 l/h, and betaine was collected from column 1. Simultaneously, 21.0 l of water was pumped to column 2 at a flow rate of 70 l/h, and a residual fraction was collected from column 2. Also simultaneously, 21.0 l of water was pumped to column 3 at flow rate of 75 l/h, and a residual fraction was collected from column 3.

Step 8: 11.0 l was circulated in the column loop, formed with column 1 at a flow rate of 45.0 l/h. Simultaneously 11.0 l was circulated in the column loop, formed with column 2 at a flow rate of 22.0 l/h. Also, simultaneously, 10.5 l was circulated in the column loop, formed with column 3, at a flow rate of 75.0 l/h.

The system and the resin ion form were balanced during 13 sequences, and the following fractions were drawn from the system: a residual fraction from columns 1, 2, 3, a recycle fraction from column 1, a sucrose product fraction from column 1 and a betaine product fraction from column 1. The results including HPLC analyses for the combined fractions are set forth in the table E1B-2 below. Table E1B-3 shows separation results calculated from step volumes, column dimensions and HPLC results.

**TABLE E1B-2**

| | Combined residual | Combined recycle | Combined sucrose | Combined betaine |
|---|---|---|---|---|
| Volume, I | 67.0 | 14.5 | 27.0 | 15.0 |
| Concentration, g/100ml | 8.8 | 14.2 | 22.0 | 5.2 |
| Sucrose % on DS | 23.5 | 79.3 | 96.0 | 20.7 |
| Betaine, % on DS | 5.9 | 3.2 | 0.6 | 36.6 |
| Others, % on DS | 70.6 | 17.5 | 3.4 | 42.7 |

**TABLE E1B-3**

| Separation parameters | |
|---|---|
| Sucrose yield, % | 78.7 |
| Betaine yield, % | 42.7 |
| Feed load, kg DS/m³ | 31.2 |
| Water/feed ratio | 5.2 |
| Sequence time, min | 94.7 |
| Product capacity, kg DS/m³h | 17.0 |

The recycle fraction needs concentration before mixing with the feed to obtain the desired feed dry substance.

### Example 1C,3-Profile/3-loop SMB separation of beet molasses - reference example

The process equipment included three columns connected in series, a feed pump, recycling pumps, an eluent water pump, heat exchangers, flow control means for the out-coming liquids as well as inlet and product valves for the various process streams. The height of all columns was 5 m and the diameter was 0.2 m. The columns were packed with a strong acid gel type cation exchange resin (Finex) in Na⁺-form. The mean bead size of the resin was about 0.36 mm. The DVB content of the resin was 5.5 %.

Before the separation, molasses was pre-coat filtered after carbonation using diatomaceous earth as a filter aid. The feed was composed as set forth below, whereby the percentages are given on a dry substance weight basis.

**TABLE E1C-1**

| Composition of the feed | |
|---|---|
| Sucrose, % on DS | 59.6 |
| Betaine, % on DS | 5.6 |
| Others (mainly salts), % on DS | 34.8 |
| Feed Concentration, g/100ml | 75.4 |

The fractionation was performed in accordance with Example 3 of US 6 093 326 by way of 5-step SMB sequence as set forth below. The aim of the separation was to separate sucrose and betaine contained therein. The feed and the eluent were used at a temperature of 75°C and ion exchanged water was used as an eluent.

Step 1: 9 l of feed was pumped to column 1 at flow rate of 35 l/h, and a recycle 1 fraction was collected from column 3. 11.0 l of feed was pumped to column 1 at a flow rate of 35 l/h, and a sucrose fraction was collected from column 3.

Step 2: 16 l of water was pumped to column 1 at a flow rate of 70 l/h, and a sucrose fraction was collected from column 3. Thereafter 4 l of water was pumped to column 1 at a flow rate of 70 l/h and a sucrose fraction was collected from column 3.

Step 3: 8 l was circulated around column 1 at a flow rate of 70 l/h, and 10 l of water was pumped to column 2 at flow rate of 40 l/h, and a betaine fraction was collected from column 3.

Step 4: 26 l of water was pumped to column 1 at a flow rate of 70 l/h, and a residual fraction was collected from column 1. 26 l of water was pumped to column 2 at a flow rate of 75 l/h, and a residual fraction was collected from column 2. 15 l of water was pumped to column 3 at flow rate of 40 l/h, and a residual fraction was collected from column 3 followed by pumping 5 l of water at a flow rate of 40 l/h to column 3 and collecting residual from column 3.

Step 5: 11 l was circulated in the loop formed around column 1 at a flow rate of 22 l/h. 12 l was circulated in the loop formed around column 2 at a flow rate of 75 l/h. 11 l was circulated in the loop formed around column 3 at a flow rate of 70 l/h.

The system and the resin ion form were balanced by repeating sequences of the above mentioned 5 steps until equilibrium had been reached, and the following fractions were drawn from the system: residual fractions from columns 1, 2, 3, a recycle fraction from column 3, a sucrose product fraction from column 3 and a betaine product fraction from column 3. The results including HPLC analyses for the combined fractions are set forth in the table E1C-2 below. Table E1C-3 shows separation results calculated from step volumes, column dimensions and HPLC results.

**TABLE E1C-2**

| | Combined residual | Combined recycle | Combined sucrose | Combined betaine |
|---|---|---|---|---|
| Volume, I | 77.0 | 13.0 | 27.0 | 15.0 |
| Concentration, g/100ml | 8.1 | | 25.2 | 7.1 |
| Sucrose % on DS | 16.7 | | 92.7 | 45.9 |
| Betaine, % on DS | 8.8 | | 0.7 | 22.9 |
| Others, % on DS | 74.5 | | 6.6 | 31.2 |

**TABLE E1C-3**

| Separation parameters | |
|---|---|
| Sucrose yield, % | 81.9 |
| Betaine yield, % | 31.7 |
| Feed load, kg DS/m³ | 32.0 |
| Water/feed ratio | 6.6 |
| Sequence time, min | 110.6 |
| Product capacity, kg DS/m³h | 15.3 |

In this example, too much recycle was taken for the dilution of molasses and therefore the recycle fraction needs concentration before mixing with the feed to obtain the desired feed concentration.

### Example 2

### Three-profile SMB separation of low green - recovery of sucrose and betaine

The process equipment includes 6 columns, required piping, a feed pump, recycling pumps, an eluent water pump, heat exchangers, feed and eluent and product tanks, flow control means for the out-coming liquids as well as inlet and product valves for the various process streams. The height of all columns is 4.0 m, the diameter of all columns is 5.15 m, and the first column is made of two compartments with an equal size. The columns are packed with a strong acid gel type cation exchange resin (Dowex monosphere 99K/350) in Na⁺-form. The mean bead size of the resin is 0.35 mm.

Before the separation, the low green is diluted with a recycle fraction (obtained from the separation) and carbonated with sodium carbonate to reduce the calcium level of the solution. Finally, the low green is pre-coat filtered using diatomaceous earth as a filtering aid.

The feed was composed as set forth below, whereby the percentages are given on a dry substance weight basis.

**TABLE E2-1**

| Composition of the feed | |
|---|---|
| Feed pH | 9.5 |
| Feed dry substance, g/100g | 50.0 |
| Sucrose, % on DS | 72.0 |
| Betaine, % on DS | 4.5 |
| Others, % on DS | 23.5 |

The fractionation is performed by way of a 10-step SMB sequence as set forth below. The feed and the eluent are used at a temperature of 80°C, and an evaporation condensate is used as an eluent.

Step 1: 8.8 m³ of feed solution is pumped into column 1 at a flow rate of 185 m³/h, and a sucrose fraction is collected from column 6.

Step 2: 6.2 m³ of feed solution is pumped into column 1 at a flow rate of 180 m³/h, and a residual fraction is collected from the same column. Simultaneously, 6.2 m³ of eluent is pumped into column 2 at a flow rate of 180 m³/h, and a betaine fraction is collected from column 3. Also simultaneously, 6.2 m³ of eluent is pumped into column 4 at a flow rate of 180 m³/h, and a betaine fraction is collected from column 5. Also simultaneously, 9.0 m³ of eluent is pumped into column 6 at a flow rate of 225 m³/h, and a sucrose fraction is collected from the same column.

Step 3: 9.0 m³ of feed solution is pumped into column 1 at a flow rate of 180 m³/h, and a residual fraction is collected from the same column. Simultaneously, 5.0 m³ of eluent is pumped into column 2 at a flow rate of 100 m³/h, and a residual fraction is collected from column 3. Also simultaneously, 5.0 m³ of eluent is pumped into column 4 at a flow rate of 100 m³/h, and a residual fraction is collected from column 5. Also simultaneously, 12.0 m³ of eluent is pumped into column 6 at a flow rate of 225 m³/h, and a sucrose fraction is collected from the same column.

Step 4: 4.0 m³ of eluent is pumped into column 6 at a flow rate of 190 m³/h, and a residual fraction is collected from column 3. Simultaneously, 4.0 m³ of eluent is pumped into column 4 at a flow rate of 190 m³/h, and a residual fraction is collected from column 5.

Step 5: 7.2 m³ is looped at a flow rate of 195 m³/h in a loop formed by all columns. There are three separation profiles in the loop.

Step 6: 15.0 m³ of eluent is pumped into column 1 at a flow rate of 190 m³/h, and a betaine fraction is collected from column 6.

Step 7: 5.0 m³ of eluent is pumped into column 1 at a flow rate of 110 m³/h, and a betaine fraction is collected from column 2. Simultaneously, 9.0 m³ of eluent is pumped into column 3 at a flow rate of 210 m³/h, and a betaine fraction is collected from column 4. Also simultaneously, 9.0 m³ of eluent is pumped into column 5 at a flow rate of 210 m³/h, and a betaine fraction is collected from column 6.

Step 8: 10.0 m³ of eluent is pumped into column 1 at a flow rate of 210 m³/h, and a residual fraction is collected from column 2. Simultaneously, 10.0 m³ of eluent is pumped into column 3 at a flow rate of 210 m³/h, and a residual fraction is collected from column 4. Also simultaneously, 10.0 m³ of eluent is pumped into column 5 at a flow rate of 210 m³/h, and a residual fraction is collected from column 6.

Step 9: 9.5 m³ of eluent is pumped into column 1 at a flow rate of 205 m³/h, and a recycle fraction is collected from column 2. Simultaneously, 6.6 m³ of is looped at a flow rate of 140 m³/h in a loop formed by columns 3 and 4. Also simultaneously, 6.6 m³ is looped at a flow rate of 140 m³/h in a loop formed by columns 5 and 6.

Step 10: 17.2 m³ is looped at a flow rate of 210 m³/h in a loop formed by columns 1 and 2. Simultaneously, 13.6 m³ is looped at a flow rate of 130 m³/h in a loop formed by columns 3 and 4. Also simultaneously, 13.6 m³ is looped at a flow rate of 130 m³/h in a loop formed by columns 5 and 6.

After equilibration of the system, residual fractions, sucrose fractions and betaine fractions are collected. The results including HPLC analyses for the combined fractions are set forth in the table below.

**TABLE E2-2**

| | Combined residual | Combined recycle | Combined sucrose | Combined betaine |
|---|---|---|---|---|
| Volume, m³ | 63.2 | 7.2 | 29.8 | 50.4 |
| Dry substance, weiqht-% | 3.7 | 15.3 | 29.9 | 2.5 |
| Dry substance, g/100ml | 3.7 | 16.3 | 33.6 | 2.5 |
| Sucrose, % on DS | 27.3 | 40.8 | 94.5 | 4.3 |
| Betaine, % on DS | 1.4 | 0.0 | 0.0 | 50.9 |
| Others, % on DS | 71.3 | 59.2 | 5.5 | 44.8 |

The overall yield calculated from these fractions is 93.2% for sucrose and 95.0% for betaine. The separation capacity for the fractionation is 54 kg dry substance per hour per m³ of the resin and the ratio of eluent water to the feed dry sustance ratio is 8.6 m³ water per ton dry substance. This corresponds to a W/F ratio of 5.3.

When the results are compared to example 1A, it can be seen that separation capacity has been improved over 40% due to a reduced sequence time (a mode with three separation profiles in a loop, a high feed load and a minimized water interval between the profiles). Furthermore, a clear improvement in the purity of the sucrose fraction and only a minor decrease in the ratio of eluent water to the feed substance and in the W/F ratio can be seen.

### Example 3 3-Profile/3-loop chromatographic SMB separation of beet molasses (low green) - recovery of sucrose and betaine

The process equipment included six columns connected in series, a feed pump, recycling pumps, an eluent water pump, heat exchangers, flow control means for the out-coming liquids as well as inlet and product valves for the various process streams. The height of all columns was 4 m and the diameter was 0.111 m. Columns consist of two parts, 2 meters each. The columns were packed with a strong acid gel type cation exchange resin (Dowex 99K/350 resin) in Na⁺-form. The mean bead size of the resin was 0.35 mm. The DVB content of the resin was approximately 6 %.

Before the separation molasses was pre-coat filtered using diatomaceous earth as a filter aid. The feed was composed as set forth below, whereby the percentages are given on a dry substance weight basis.

**TABLE E3-1**

| Composition of low green | |
|---|---|
| Sucrose, % on DS | 70.4 |
| Betaine, % on DS | 5.1 |
| Trisaccharides, % on DS | 2.6 |
| Disaccharides, % on DS | 0.1 |
| Glucose, % on DS | 0.2 |
| Fructose, % on DS | 0.3 |
| Inositol, % on DS | 0.2 |
| Glycerol, % on DS | 0.1 |
| Others (mainly salts), % on DS | 21.0 |

**TABLE E3-2**

| Composition of the feed | |
|---|---|
| Sucrose, % on DS | 66.8 |
| Betaine, % on DS | 5.4 |
| Trisaccharides, % on DS | 3.5 |
| Others( mainly salts), % on DS | 24.3 |
| Conductivity, ms/cm | 13.5 |
| Feed concentration, g/100ml | 61.2 |

The fractionation was performed by way of an 11-step SMB sequence as set forth below. The aim of the separation was to separate sucrose and betaine contained therein. The feed and the eluent were used at a temperature of 80°C and ion exchanged water was used as an eluent.

Step 1: 6.3 l of feed was pumped to column 1 at flow rate of 55 l/h and sucrose fraction was collected from column 6 (the last column).

Step 2: 2.4 l of feed was pumped to column 1 at flow rate of 34 l/h, and a residual fraction was collected from column 1. Simultaneously, 2.0 l water was pumped to column 2 at a flow rate of 28.5 l/h and a betaine fraction was collected from column 3. Also simultaneously, 2.8 l of water was pumped to column 4 at a flow rate of 40 l/h, and a betaine fraction was collected from column 5. Also simultaneously 5.8 l of water was pumped to column 6 at a flow rate of 85 l/h, and sucrose was collected from column 6 (the last column).

Step 3: 2.5 l of feed was pumped to column 1 at flow rate of 42 l/h, and residual fraction was collected from column 1. Simultaneously, 5.1 l water was pumped to column 2 at a flow rate of 85 l/h, and residual fraction was collected from column 3. Also simultaneously, 3.6 l of water was pumped to column 4 at a flow rate of 60 l/h, and a sucrose fraction was collected from column 4. Also simultaneously, 1.5 l water was pumped to column 6 at a flow rate of 25 l/h, and a betaine fraction was collected from column 5.

Step 4: 2.5 l of feed was pumped to column 1 at flow rate of 47 l/h, and a residual fraction was collected from column 1. Simultaneously, 3.1 l water was pumped to column 2 at a flow rate of 58 l/h, and recycle fraction was collected from column 3. Also simultaneously, 3.7 l of water was pumped to column 4 at a flow rate of 70 l/h, and a sucrose fraction was collected from column 4. Also simultaneously 1.0 l water was pumped to column 6 at a flow rate of 19 l/h, and a betaine fraction was collected from column 5.

Step 5: 0.4 l of water was pumped to column 6 at flow rate of 10.5 l/h, and a residual fraction was collected from column 1. Simultaneously, 3.2 l water was pumped to column 2 at a flow rate of 85 l/h, and recycle fraction was collected from column 3. Also simultaneously, 2.2 l of water was pumped to column 4 at a flow rate of 58 l/h, and a residual fraction was collected from column 5.

Step 6: 6.8 l was circulated in the column loop, formed with columns 1, 2, 3, 4, 5 and 6 at a flow rate of 70 l/h.

Step 7: 3.3 l of water was pumped to column 1 at a flow rate of 70 l/h and a PART fraction was collected from column 4. Simultaneously, 3.3 l was circulated in the column loop, formed with columns 5 and 6, at a flow rate of 70 l/h.

Step 8: 1.0 l of water was pumped to column 1 at flow rate of 70 l/h, and a betaine fraction was taken from column 6.

Step 9: 2.0 l of water was pumped to column 1 at a flow rate of 22,5 l/h and a residual was collected from column 2. Simultaneously 5.4 of water was pumped to column 3 at a flow rate of 61 l/h and a betaine fraction was collected from column 4. Also simultaneously 6.5 l of water was pumped to column 5 at a flow rate of 73 l/h and betaine fraction was collected from column 6.

Step 10: 9.5 l of water was pumped to column 1 at a flow rate of 80 l/h and a residual was collected from column 2. Simultaneously, 3.3 of water was pumped to column 3 at a flow rate of 28 l/h, and a residual fraction was collected from 4. Also simultaneously, 2.3 l of water was pumped to column 5 at a flow rate of 73 l/h, and residual fraction was collected from column 6.

Step 11: 5.0 l was circulated in the column loop, formed with columns 1 and 2 at a flow rate of 45.5 l/h. Simultaneously, 5.0 l was circulated in the column loop, formed with columns 3 and 4, at a flow rate of 45.5 l/h. Also Simultaneously, 5.7 l was circulated in the column loop, formed with columns 5 and 6, at a flow rate of 52 l/h.

The system and the resin ion form were balanced with 10-18 sequences and the following fractions were drawn from the system: a residual fraction from columns 1, 2, 3, 4, 5 and 6, a recycle fraction from column 3, sucrose product fractions from columns 4 and 6 and betaine product fractions from columns 3, 4, 5 and 6. The results including HPLC analyses for the combined fractions are set forth in Table E3-3. In the test run PART fraction was collected from column 4. The PART feeding step was used so that the PART fraction did not reduce the purities of the sucrose or betaine fractions. Table E3-4 shows the separation results calculated from step volumes, column dimensions and HPLC results.

**TABLE E3-3**

| | Combined residual | Combined recycle | Sucrose column 4 | Sucrose column 6 | Combined sucrose | Combined betaine |
|---|---|---|---|---|---|---|
| Volume, l | 33.5 | 6.3 | 7.3 | 12.1 | 19.4 | 18.8 |
| Concentration, g/100ml | 5.0 | 12.1 | 36.1 | 22.6 | 27.7 | 3.9 |
| Sucrose % on DS | 13.3 | 36.0 | 95.2 | 93.5 | 94.6 | 7.2 |
| Betaine, % on DS | 3.2 | 7.5 | 0.1 | 0.0 | 0.0 | 46.0 |
| Others, % on DS | 83.5 | 56.5 | 4.7 | 6.5 | 5.4 | 46.8 |

**TABLE E3-4**

| Separation parameters | |
|---|---|
| Sucrose yield, % | 94.9 |
| Betaine yield, % | 86.0 |
| Feed load, kg DS/m³ | 36.7 |
| Water/Feed ratio, - | 4.7 |
| Sequence time, min | 48.7 |
| Product capacity, kg DS/m³h | 41.2 |

Due to the flow rate limitations in pilot scale, in plant scale it is possible to run the process much faster which would give a capacity of 56 - 67 kg DS/m³h.

Calculation of where recycled PART dry substance ended up was done assuming that the dry substance has a void of 56% bed volume since it in this case part is mainly betaine. Also band broadening was taken into account empirically. The recycled PART movement was calculated by subtracting volume of a step from column void starting with step were eluent was replaced by eluent replacement solution. It was calculated that PART will leave the system in recycle fraction. This method was very accurate for the PART profile front movement.

### Example 4 3-loop/3-profile SMB separation of Mg²⁺ sulphite spent liquor (MgSSL) - recovery of xylose

The process equipment includes six columns connected in series, a feed pump, recycling pumps, an eluent water pump, heat exchangers, required piping, required tanks, flow control means for the out-coming liquids as well as inlet and product valves for the various process streams. The height of all columns is 3.3 m and each column has a diameter of 0.11 m. The columns are packed with a strong acid gel type cation exchange resin (manufactured by Finex) in Mg²⁺-form. The divinylbenzene content of the resin is 6.5% and the mean bead size of the resin is 0.38 mm.

Before the separation, Mg²⁺ sulphite spent liquor is pre-coat filtered by using Arbocel B800 as a filter aid. The feed dry substance is then adjusted with ion exchanged water to 48g/100g and the feed liquor pH is 3.1. The feed is composed as set forth below, whereby the percentages are given on a dry substance weight basis.

**TABLE E4-1**

| Composition of the feed | |
|---|---|
| Glucose, % on DS | 2.1 |
| Xylose, % on DS | 15.2 |
| Galactose+rhamnose, % on DS | 1.7 |
| Arabinose+mannose, % on DS | 1.7 |
| Others, % on DS | 79.3 |

The fractionation is performed by way of a 12-step SMB sequence as set forth below. The aim of the separation is to separate xylose contained therein. The feed and the eluent are used at a temperature of 65°C and ion exchanged water is used as an eluent.

Step 1: 1.4 l of feed solution is pumped into column 1 at a flow rate of 77 l/h, and a recycle fraction is collected from column 2. Simultaneously 1.4 l is circulated in the column loop, formed with columns 3 and 4, at a flow rate of 77 l/h. Also simultaneously, 1.4 l is circulated in the column loop, formed with columns 5 and 6, at a flow rate of 77 l/h.

Step 2: 1.7 l of feed solution is pumped into the first column at a flow rate of 63 l/h, and a recycle fraction is collected from column 4. Simultaneously, 1.4 l is circulated in the column loop, formed with columns 5 and 6, at a flow rate of 49 l/h.

Step 3: 2.4 l of feed solution is pumped into column 1 at a flow rate of 56 l/h, and a recycle fraction is collected from column 6 (the last column).

Step 4: 1.4 l of feed solution is pumped into column 1 at a flow rate of 63 l/h, and a residual fraction is collected from the same column. Simultaneously 1.4 l of eluent is pumped into column 2 at a flow rate of 63 l/h, and a xylose fraction is collected from column 6.

Step 5: 4.9 l of feed solution is pumped into column 1 at a flow rate of 63 l/h and a residual fraction is collected from the same column. Simultaneously, 4.2 l of eluent is pumped into column 2 at a flow rate of 56 l/h, and a residual fraction is collected from column 3. Also simultaneously, 2.8 l of eluent is pumped into column 4 at a flow rate of 38 l/h, and a residual fraction is collected from column 5. Also simultaneously, 2.8 l of eluent is pumped into column 6 at a flow rate of 38 l/h, and a xylose fraction is collected from the same column.

Step 6: 2.1 l of feed solution is pumped into column 1 at a flow rate of 63 l/h and a residual fraction is collected from column 3. Simultaneously, 2.8 l of eluent is pumped into column 4 at a flow rate of 84 l/h, and a residual fraction is collected from column 5. Also simultaneously, 2.4 l of eluent is pumped into column 6 at a flow rate of 73 l/h, and a xylose fraction is collected from the same column.

Step 7: 1.0 l of feed solution is pumped into the first column at a flow rate of 59 l/h, and a xylose fraction is collected from column 6.

Step 8: 1.0 l of feed solution is pumped into column 1 at a flow rate of 59 l/h, and a recycle fraction is collected from column 6.

Step 9: 7.0 l is circulated in the column loop, formed with columns 1 and 2, at a flow rate of 59 l/h. Simultaneously, 8.7 l is circulated in the column loop, formed with columns 3 and 4, at a flow rate of 77 l/h. Also simultaneously, 8.4 l is circulated in the column loop, formed with columns 5 and 6, at a flow rate of 73 l/h.

Step 10: 4.2 l of eluent is pumped into the first column at a flow rate of 80 l/h and a residual fraction is collected from column 2. Simultaneously, 4.2 l of eluent is pumped into column 3 at a flow rate of 80 l/h and a residual fraction is collected from column 4. Also simultaneously, 4.2 l of eluent is pumped into column 5 at a flow rate of 80 l/h and a residual fraction is collected from column 6.

Step 11: 1.4 l of eluent is pumped into column 5 at a flow rate of 77 l/h, and a residual fraction is collected from column 2. Simultaneously, 1.4 l of eluent is pumped into column 3 at a flow rate of 77 l/h, and a residual fraction is collected from column 4.

Step 12: 4.9 l is circulated in the column loop, formed with columns 1 and 2, at a flow rate of 84 l/h. Simultaneously, 3.5 l is circulated in the column loop, formed with columns 3 and 4, at a flow rate of 59 l/h. Also simultaneously, 4.2 l is circulated in the column loop, formed with columns 5 and 6, at a flow rate of 73 l/h.

After equilibration of the system by repeating the separation sequence 10 to 18 times, the following fractions are drawn from the system: a residual fraction from all columns, recycle fractions from columns 2, 4 and 6 and xylose product fractions from column 6 (the last column). The results including HPLC analyses for the combined residual, recycle and xylose fractions are set forth in the table below.

**TABLE E4-2**

| | Combined residual | Combined recycle | Combined xylose |
|---|---|---|---|
| Volume, I | 33.4 | 6.6 | 7.7 |
| Dry substance, g/100ml | 18.0 | 25.8 | 23.5 |
| Glucose, % on DS | 1.1 | 2.9 | 4.6 |
| Xylose, % on DS | 1.2 | 24.5 | 52.9 |
| Galactose+rhamnose, % on DS | 0.1 | 3.1 | 5.6 |
| Arabinose+mannose, % on DS | 0.3 | 2.6 | 5.6 |
| Others, % on DS | 97.3 | 66.9 | 31.3 |

The overall xylose yield calculated from these fractions is 93%. The sequence time for the separation in the example is only 30.3 minutes and separation capacity calculated from the combined residual and xylose fractions is over 80 kg/h/m³. W/F (water to feed, vol/vol) ratio for the separation is 2.0.

## Claims

1. A process of separating and recovering at least one product from a feed solution containing at least one product compound selected from sugars, sugar alcohols, sugar acids, organic acids and betaine, whereby the process is carried out in a chromatographic sequential simulated moving bed (SMB) system, which comprises a plurality of columns containing one or more partial packed beds, wherein the columns form one or more loops, comprising
creating three successive separation profiles in the system by repeating a predetermined separation sequence, whereby said three successive separation profiles are simultaneously present in the system, and each separation profile comprises at least one product subprofile, a residual subprofile and optionally other subprofiles,
moving said three separation profiles forward through the system by repeating the predetermined separation sequence, and
recovering at least one product fraction enriched in one product compound from a column and also recovering at least one further fraction from one or more other columns of the system, whereby said at least one further fraction is a further product fraction enriched in the same product compound and/or a recycle fraction containing the same product compound.

2. A process as claimed in claim 1, wherein said at least one product fraction enriched in one product compound and said at least one further fraction are recovered from one and the same separation profile during more than one separation sequences or
from more than one separation profiles during one and the same separation sequence.

3. A process as claimed in claim 1, wherein said at least one product fraction enriched in one product compound is recovered from the last column of the system.

4. A process as claimed in claim 1, wherein said one or more other columns are selected from any upstream columns of the system in regard to the column from which said at least one product fraction is recovered.

5. A process as claimed in claim 1, wherein said three separation profiles are present in a loop formed by all columns of the system.

6. A process as claimed in claim 1, wherein said plurality of columns form three separate loops and a separation profile is simultaneously moved forward in each loop.

7. A process as claimed in claim 1, wherein the process further comprises recovering one or more further fractions enriched in at least one further product compound.

8. A process as claimed in claims 1 and 7, wherein said one product compound is a sugar selected from sucrose and said further product compound is betaine.

9. A process as claimed in claim 8, wherein the process comprises recovering at least one sucrose fraction from a column and at least one further sucrose fraction from one or more other columns of the system and also recovering at least one betaine fraction from any columns of the system.

10. A process as claimed in claim 1, wherein the sugar is xylose.

11. A process as claimed in claim 10, wherein the process comprises recovering at least one xylose fraction from a column and also recovering at least one further xylose fraction and/or at least one recycle fraction containing xylose from one or more other columns of the system.

12. A process as claimed in claim 1, wherein said feed solution is selected from sugar beet based solutions, preferably low green and molasses, and wood hydrolysates, preferably spent sulphite pulping liquor.

13. A process as claimed in claim 1 for recovering sucrose and betaine from a sugar beet based solution in a chromatographic sequential simulated moving bed (SMB) system, which comprises a plurality of columns containing one or more partial packed beds, wherein the columns form one or more loops, comprising
creating three successive separation profiles in the system by repeating a predetermined separation sequence, whereby said three successive separation profiles are simultaneously present in the system, and each separation profile comprises a sucrose subprofile, a betaine subprofile, a residual subprofile and optionally other subprofiles,
moving said three separation profiles forward through the system by repeating the predetermined separation sequence, and
recovering at least one sucrose fraction from a column and at least one further sucrose fraction from one or more other columns of the system and also recovering one or more betaine fractions from any columns of the system.

14. A process as claimed in claim 13, wherein the sugar beet based solution is selected from low green, molasses, thick juice and raw juice.

15. A process as claimed in claim 13, wherein the sucrose content of the sucrose fractions is more than 90%, preferably more than 92% and more preferably more than 94% on the dry substance (DS),
and wherein the dry substance amount of said further sucrose fraction recovered is more than 10%, preferably more than 30% and more preferably more than 50%, based on the dry substance amount of the combined sucrose fractions recovered,
wherein the process provides to combined sucrose fractions a sucrose yield of more than 90%, preferably more than 92% and more preferably more than 94% based on the sucrose of the feed solution,
wherein the betaine content of the betaine fractions is more than 35%, preferably more than 45% and more preferably more than 55% on DS and
wherein the yield of betaine to the betaine fraction(s) is more than 85%, preferably more than 92% and more preferably more than 94%.

16. A process as claimed in claim 1 for recovering xylose from a plant-based hydrolysate in a chromatographic sequential simulated moving bed system, which comprises a plurality of columns containing one or more partial packed beds, wherein the columns form one or more loops, comprising
creating three successive separation profiles in the system by repeating a predetermined separation sequence, whereby said three successive separation profiles are simultaneously present in the system, and each separation profile comprises a xylose subprofile, a residual subprofile and optionally other subprofiles,
moving said three separation profiles forward through the system by repeating the predetermined separation sequence, and
recovering at least one xylose fraction from a column and also recovering at least one further xylose fraction and/or at least one recycle fraction containing xylose from one or more other columns of the system.

17. A process as claimed in claim 16, wherein the plant-based hydrolysate is a hemicellulose hydrolysate, preferably spent sulphite pulping liquor.

18. A process as claimed in claim 16, wherein the xylose content of the xylose fraction(s) is more than 45%, preferably more than 50% and more preferably more than 55% on DS, and
wherein the process provides a xylose yield of more than 85%, preferably more than 90% and more preferably more than 93% on the xylose of the feed solution.

19. A process as claimed in claim 1, wherein the process further comprises introducing one or more parts of said three separation profiles back to one or more eluent introduction positions of the system to substitute a portion of the eluent, wherein said parts comprise components selected from product compounds and residual components.

## Patentansprüche

1. Ein Verfahren zur Trennung und Rückgewinnung mindestens eines Produkts aus einer Feed-Lösung, enthaltend mindestens eine Produktverbindung ausgewählt aus Zuckern, Zuckeralkoholen, Zuckersäuren, organischen Säuren und Betain, wobei das Verfahren in einem chromatographischen sequenziellen Simulated Moving Bed (SMB) System ausgeführt wird, das eine Vielzahl von Säulen, enthaltend ein oder mehr Teilschüttschichten, umfasst, wobei die Säulen ein oder mehr Windungen ausbilden, umfassend
Erzeugen dreier aufeinanderfolgender Trennprofile im System durch Wiederholen einer vorgegebenen Trennsequenz, wobei die drei aufeinanderfolgenden Trennprofile simultan im System vorhanden sind und jedes Trennprofil mindestens ein Produkt-Teilprofil, ein Rückstands-Teilprofil und optional weitere Teilprofile umfasst,
Vorwärtsbewegen der drei Trennprofile durch das System durch Wiederholen der vorgegebenen Trennsequenz und
Rückgewinnen mindestens einer mit einer Produktverbindung angereicherten Produktfraktion von einer Säule und ferner Rückgewinnen von mindestens einer weiteren Fraktion von ein oder mehr weiteren Säulen des Systems, wobei die mindestens eine weitere Fraktion eine weitere mit derselben Produktverbindung angereicherte Produktfraktion und/oder eine Recyclingfraktion enthaltend dieselbe Produktverbindung ist.

2. Verfahren gemäß Anspruch 1, wobei die mindestens eine mit einer Produktverbindung angereicherte Produktfraktion und die mindestens eine weitere Produktfraktion von ein und demselben Trennprofil während mehr als einer Trennsequenz zurückgewonnen werden.

3. Verfahren gemäß Anspruch 1, wobei die mindestens eine mit einer Produktverbindung angereicherte Produktfraktion von der letzten Säule des Systems zurückgewonnen wird.

4. Verfahren gemäß Anspruch 1, wobei die ein oder mehr weiteren Säulen ausgewählt sind aus beliebigen vorgeschalteten Säulen des Systems bezogen auf die Säule von der die mindestens eine Produktfraktion zurückgewonnen wird.

5. Verfahren gemäß Anspruch 1, wobei die drei Trennprofile in einer Windung, die durch alle Säulen des Systems gebildet wird, vorhanden sind.

6. Verfahren gemäß Anspruch 1, wobei die Vielzahl an Säulen drei getrennte Windungen bildet und ein Trennprofil simultan durch jede Windung vorwärts bewegt wird.

7. Verfahren gemäß Anspruch 1, wobei das Verfahren ferner das Rückgewinnen einer oder mehr mit mindestens einer weiteren Produktverbindung angereicherten Fraktionen umfasst.

8. Verfahren gemäß einem der Ansprüche 1 bis 7, wobei die eine Produktverbindung ein Zucker, ausgewählt aus Saccharose, und die eine weitere Produktverbindung Betain ist.

9. Verfahren gemäß Anspruch 8, wobei das Verfahren das Rückgewinnen mindestens einer Saccharose-Fraktion von einer oder mehr Säulen des Systems und ferner das Rückgewinnen mindestens einer Betain-Fraktion von beliebigen Säulen des Systems umfasst.

10. Verfahren gemäß Anspruch 1, wobei der Zucker Xylose ist.

11. Verfahren gemäß Anspruch 10, wobei das Verfahren das Rückgewinnen mindestens einer Xylose-Fraktion von einer Säule und ferner das Rückgewinnen von mindestens einer weiteren Xylose-Fraktion und/oder mindestens einer Xylose enthaltenden Recyclingfraktion von einer oder mehr weiteren Säulen des Systems umfasst.

12. Verfahren gemäß Anspruch 1, wobei die Feed-Lösung ausgewählt ist aus Rübenzucker-basierten Lösungen, vorzugsweise "low green" und Melasse, und Holzhydrolysate, vorzugsweise beim Sulfit-Verfahren anfallende Schwarzlauge.

13. Ein Verfahren gemäß Anspruch 1 zur Rückgewinnung von Saccharose und Betain von einer Rübenzucker-basierten Lösung in einem chromatographischen sequenziellen Simulated Moving Bed (SMB) System, das eine Vielzahl von Säulen, enthaltend eine oder mehr Teilschüttschichten, umfasst, wobei die Säulen ein oder mehr Windungen ausbilden, umfassend
Erzeugen dreier aufeinanderfolgender Trennprofile im System durch Wiederholen einer vorgegebenen Trennsequenz, wobei die drei aufeinanderfolgenden Trennprofile simultan im System vorhanden sind und jedes Trennprofil mindestens ein Saccharose-Teilprofil, ein Betain-Teilprofil, ein Rückstands-Teilprofil und optional weitere Teilprofile umfasst,
Vorwärtsbewegen der drei Trennprofile durch das System durch Wiederholen der vorgegebenen Trennsequenz und
Rückgewinnen mindestens einer Saccharose-Fraktion von einer Säule und mindestens einer weiteren Saccharose-Fraktion von ein oder mehr weiteren Säulen des Systems und ferner Rückgewinnen von einer oder mehr Betain-Fraktionen von beliebigen Säulen des Systems.

14. Verfahren gemäß Anspruch 13, wobei die Rübenzucker-basierte Lösung ausgewählt ist aus "low green", Melasse, Dicksaft und Rohsaft.

15. Verfahren nach Anspruch 13, wobei der Saccharosegehalt der Saccharose-Fraktionen mehr als 90%, vorzugsweise mehr als 92% und besonders bevorzugt mehr als 94% der Trockensubstanz beträgt,
und wobei die zurückgewonnene Menge an Trockensubstanz der weiteren Saccharose-Fraktion mehr als 10%, vorzugsweise mehr als 30% und besonders bevorzugt mehr als 50%, basierend auf der Menge an Trockensubstanz der kombinierten zurückgewonnenen Saccharose-Fraktionen, beträgt,
wobei das Verfahren für die kombinierten Saccharose-Fraktionen eine Saccharoseausbeute von mehr als 90%, vorzugsweise mehr als 92% und besonders bevorzugt mehr als 94%, basierend auf der Saccharose der Feed-Lösung, liefert,
wobei der Betaingehalt der Betain-Fraktionen mehr als 35%, vorzugsweise mehr als 45% und besonders bevorzugt mehr als 55% der Trockensubstanz beträgt, und
wobei die Betainausbeute an der/den Betain-Fraktion/en mehr als 85%, vorzugsweise mehr als 92% und besonders bevorzugt mehr als 94% beträgt.

16. Verfahren gemäß Anspruch 1 zur Rückgewinnung von Xylose aus einem Pflanzen-basierten Hydrolysat in einem chromatographischen sequenziellen Simulated Moving Bed (SMB) System, das eine Vielzahl von Säulen, enthaltend eine oder mehr Teilschüttschichten, umfasst, wobei die Säulen ein oder mehr Windungen ausbilden, umfassend
Erzeugen dreier aufeinanderfolgender Trennprofile im System durch Wiederholen einer vorgegebenen Trennsequenz, wobei die drei aufeinanderfolgenden Trennprofile simultan im System vorhanden sind und jedes Trennprofil mindestens ein Xylose-Teilprofil, ein Rückstands-Teilprofil und optional weitere Teilprofile umfasst,
Vorwärtsbewegen der drei Trennprofile durch das System durch Wiederholen der vorgegebenen Trennsequenz und
Rückgewinnen mindestens einer Xylose-Fraktion von einer Säule und ferner Rückgewinnen mindestens einer weiteren Xylose-Fraktion und/oder mindestens einer Xylose enthaltenden Recyclingfraktion von ein oder mehr weiteren Säulen des Systems.

17. Verfahren gemäß Anspruch 16, wobei das Pflanzen-basierte Hydrolysat ein Hemicellulose-Hydrolysat, vorzugsweise beim Sulfit-Verfahren anfallende Schwarzlauge, ist.

18. Verfahren gemäß Anspruch 16, wobei der Xylosegehalt der Xylose-Fraktion/en mehr als 45%, vorzugsweise mehr als 50% und besonders bevorzugt mehr als 55% der Trockensubstanz beträgt, und
wobei das Verfahren eine Xyloseausbeute von mehr als 85%, vozugsweise mehr als 90% und besonders bevorzugt mehr als 93%, basierend auf der Xylose der Feed-Lösung, liefert.

19. Verfahren gemäß Anspruch 1, wobei das Verfahren ferner die Rückführung von ein oder mehr Bestandteilen der drei Trennprofile in eine oder mehr Eluent-Einführungspositionen des Systems umfasst, um einen Anteil des Eluenten zu ersetzen, wobei die Bestandteile Komponenten, ausgewählt aus Produktverbindungen und Rückstandsverbindungen, umfassen.

## Revendications

1. Procédé de séparation et de récupération d'au moins un produit à partir d'une solution d'alimentation contenant au moins un composé de produit sélectionné à partir de sucres, d'alcools de sucre, d'acides de sucre, d'acides organiques et de bétaïne, de sorte que le procédé est mis en oeuvre dans un système de chromatographie à lit mobile simulé séquentiel (SMB), qui comprend plusieurs colonnes contenant un ou plusieurs lits partiels tassés, dans lequel les colonnes forment une ou plusieurs boucles, comprenant
la création de trois profils de séparation successifs dans le système en répétant une séquence de séparation prédéterminée, de sorte que les trois profils de séparation successifs sont simultanément présents dans le système, et chaque profil de séparation comprend au moins un sous-profil de produit, un sous-profil résiduel et, facultativement, d'autres sous-profils,
le déplacement des trois profils de séparation vers l'avant à travers le système en répétant la séquence de séparation prédéterminée, et
la récupération d'au moins une fraction de produit enrichi en composé de produit à partir d'un colonne et également la récupération d'au moins une fraction supplémentaire provenant d'une ou plusieurs autres colonnes du système, de sorte que la au moins une fraction supplémentaire est une fraction supplémentaire de produit enrichi en ce même composé de produit et/ou une fraction de recyclage contenant le même composé de produit.

2. Procédé selon la revendication 1, dans lequel la au moins une fraction supplémentaire de produit enrichi en un composé de produit et la au moins une fraction supplémentaire sont récupérées à partir d'un seul et même profil de séparation au cours de plus d'une séquence de séparation ou
à partir de plus d'un profil de séparation au cours d'une seule et même séquence de séparation.

3. Procédé selon la revendication 1, dans lequel la au moins une fraction de produit enrichi en un composé de produit est récupérée à partir de la dernière colonne du système.

4. Procédé selon la revendication 1, dans lequel les une ou plusieurs autres colonnes sont sélectionnées à partir de n'importe quelle colonne du système en amont par rapport à la colonne à partir de laquelle la au moins une fraction de produit est récupérée.

5. Procédé selon la revendication 1, dans lequel les trois profils de séparation sont présents dans une boucle formée par toutes les colonnes du système.

6. Procédé selon la revendication 1, dans lequel les plusieurs colonnes forment trois boucles séparées et un profil de séparation est déplacé vers l'avant simultanément dans chaque boucle.

7. Procédé selon la revendication 1, dans lequel le procédé comprend en outre la récupération d'une ou plusieurs fractions supplémentaires enrichies en au moins un composé de produit supplémentaire.

8. Procédé selon les revendications 1 et 7, dans lequel le un composé de produit est un sucre choisi parmi le saccharose et le composé de produit supplémentaire est de la bétaïne.

9. Procédé selon la revendication 8, dans lequel le procédé comprend la récupération d'au moins une fraction de saccharose à partir d'une colonne et au moins une fraction supplémentaire de saccharose provenant d'une ou plusieurs autres colonnes du système et également la récupération d'au moins une fraction de bétaïne à partir de n'importe quelle colonne du système.

10. Procédé selon la revendication 1, dans lequel le sucre est du xylose.

11. Procédé selon la revendication 10, dans lequel le procédé consiste à récupérer au moins une fraction de xylose à partir d'une colonne et à récupérer également au moins une fraction de xylose supplémentaire et/ou au moins une fraction de recyclage contenant du xylose à partir d'une ou plusieurs autres colonnes du système.

12. Procédé selon la revendication 1, dans lequel la solution d'alimentation est choisie parmi des solutions à base de betterave à sucre, de préférence de bas vert et de mélasse , et d'hydrolysats du bois, de préférence une liqueur de pâte au sulfite épuisée.

13. Procédé selon la revendication 1 pour récupérer du saccharose et de la bétaïne à partir d'une solution à base de betterave à sucre dans un système de chromatographie à lit mobile simulé séquentiel (SMB), qui comprend plusieurs colonnes contenant un ou plusieurs lits partiels tassés, dans lequel les colonnes forment une ou plusieurs boucles, comprenant
la création de trois profils de séparation successifs dans le système en répétant une séquence de séparation prédéterminée, de sorte que les trois profils de séparation successifs sont simultanément présents dans le système, et chaque profil de séparation comprend au moins un sous-profil de produit, un sous-profil de résidu et, facultativement, d'autres sous-profils,
le déplacement des trois profils de séparation vers l'avant à travers le système en répétant la séquence de séparation prédéterminée, et
la récupération d'au moins une fraction de saccharose à partir d'un colonne et d'au moins une fraction supplémentaire provenant d'une ou plusieurs autres colonnes du système et également la récupération d'une ou plusieurs fractions de bétaïne à partir de n'importe quelle colonne du system.

14. Procédé selon la revendication 13, dans lequel la solution à base de betterave à sucre est choisie parmi du bas vert, de la mélasse, un jus épais et un jus brut.

15. Procédé selon la revendication 13, dans lequel la teneur en saccharose des fractions de saccharose est supérieure à 90 %, de préférence supérieure à 92 % et plus préférentiellement supérieure à 94 % par rapport à la substance sèche (DS),
et dans lequel la quantité de substance sèche de la fraction de saccharose supplémentaire récupérée est supérieure à 10 %, de préférence supérieure à 30 % et plus préférentiellement supérieure à 50 %, sur la base de la quantité de matière sèche des fractions de saccharose combinées récupérées,
dans lequel le procédé donne aux fractions de saccharose combinées un rendement en saccharose supérieur à 90 %, de préférence supérieur à 92 % et plus préférentiellement supérieur à 94 % sur la base du saccharose de la solution d'alimentation,
dans lequel la teneur en bétaïne des fractions de bétaïne est supérieure à 35 %, de préférence supérieure à 45 %, et plus préférentiellement supérieure à 55 % par rapport à DS et
dans lequel le rendement en bétaïne de la fraction de bétaïne ou des fractions de bétaïne est supérieur à 85 %, de préférence supérieur à 92 % et plus préférentiellement supérieur à 94 %.

16. Procédé selon la revendication 1 pour récupérer du xylose à partir d'un hydrolysat à base de plante dans un système de chromatographie à lit mobile simulé séquentiel, qui comprend plusieurs colonnes contenant un ou plusieurs lits partiels tassés, dans lequel les colonnes forment une ou plusieurs boucles , comprenant
la création de trois profils de séparation successifs dans le système en répétant une séquence de séparation prédéterminée, de sorte que les trois profils de séparation successifs sont simultanément présents dans le système, et chaque profil de séparation comprend un sous-profil de xylose, un sous-profil de résidu et, facultativement, d'autres sous-profils,
le déplacement des trois profils de séparation vers l'avant à travers le système en répétant la séquence de séparation prédéterminée, et
la récupération d'au moins une fraction de xylose à partir d'un colonne et également la récupération d'au moins une fraction de xylose supplémentaire et/ou une fraction de recyclage contenant du xylose à partir d'une ou plusieurs autres colonnes du système.

17. Procédé selon la revendication 16, dans lequel l'hydrolysat à base de plante est un hydrolysat d'hémicellulose, de préférence une liqueur de pâte au sulfite épuisée.

18. Procédé selon la revendication 16, dans lequel la teneur en xylose de la fraction ou des fractions de xylose est supérieure à 45 %, de préférence supérieure à 50 %, et plus préférentiellement supérieure à 55 % par rapport à DS, et
dans lequel le procédé fournit un rendement en xylose supérieur à 85 %, de préférence supérieur à 90 % et plus préférentiellement supérieur à 93 % par rapport au xylose de la solution d'alimentation.

19. Procédé selon la revendication 1, dans lequel le procédé comprend en outre l'introduction d'une ou plusieurs parties des trois profils de séparation en retour vers une ou plusieurs positions d'introduction d'éluant du système pour remplacer une partie de l'éluant, dans lequel les parties comprennent des composants choisis parmi des composés de produit et des composants résiduels.
